# EUROPEAN PATENT SPECIFICATION

(11) **EP 0 462 101 B1**
(45) Date of publication and mention of the grant of the patent: **13.07.1994**
(21) Application number: 89903614.9
(22) Date of filing: 09.03.1989
(51) Int. Cl.: A61K 7/06

(54) **COMPOSITION FOR TREATING PEDICULOSIS CAPITIS**
MITTEL ZUR BEHANDLUNG VON PEDICULOSIS CAPITIS
COMPOSE POUR TRAITER LA PEDICULOSE DE LA TETE

(43) Date of publication of application: 27.12.1991
(73) Proprietor: GENDERM CORPORATION, Lincolnshire Illinois 60069 (US)
(72) Inventor: BERNSTEIN Joel E., Deerfield, IL 60015 (US)
(74) Representative: Woodman, Derek
(86) International application number: US8900944
(87) International publication number: WO9010432

(56) References cited:
- EP-A- 0 287 805
- DE-A- 2 637 576
- DE-A- 3 605 287
- GB-A- 2 098 068

## Description

### Background of the Invention

The present application relates generally to the treatment of parasite infestations and, more particularly, to methods and composition to treat lice infections.

Pediculosis capitis (head lice ) is an infestation of Pediculosis humanus, the human louse. This insect is transmitted to noninfected individuals through shared clothing and hairbrushes. Once present on the head, the adult female louse has a lifespan of about 25 days and lays up to 10 eggs each day which are attached to the hair shaft with a strong cement. Eggs hatch within 7-9 days producing more adult lice which continue the cycle. As the lice feed, they inject their digestive juices and fecal material into the skin and these "bites" cause pruritus.

In the past, chemical agents, including insecticides, have been used to treat pediculosis. These compounds include cholinesterase inhibitors such as neostigmine (Merck Index, Tenth Edition, entry no. 6311) or prostigmine (Merck Index, Tenth Edition, entry no. 6311), and range from fairly toxic (the pyrethrins, Merck Index, Tenth Edition, entry no. 7865) to very toxic (lindane, Merck Index, Tenth Edition, entry no. 5329), malathion (Merck Index, Tenth Edition, entry no. 5522). All methods involve the exposure of the scalp and hair to these agents in shampoos or creams which are washed off relatively quickly.

While these current treatments are generally effective at killing the adult lice and eggs (nits), the dead nits remain firmly attached to the hairs after treatment. This is unsettling to the patient, as well a significant cosmetic problem. The only current means of removing dead nits is with a fine-toothed comb or forceps. These methods are time consuming and less than certain.

Formic acid, CH₂O₂, is an acid produced by ants through a distillation process. It can also be made by heating carbon monoxide and NaOH under pressure and decanting the resulting sodium formate with sulfuric acid (H₂SO₄). Formic acid is a colorless liquid that is irritating to the skin. It has, however, been used in cosmetics (0.05% - .2%) as a preservative or an astringent.

It has been found, surprisingly, that formic acid is useful in the treatment of pediculosis in that concentrations of formic acid from about 1% to about 25% can be used to remove dead nits from hair without producing undue damage to hair or skin. This "nit removing" activity of formic acid can be observed and utilized clinically either by incorporating formic acid by itself into a shampoo or rinse for use after a pediculicide preparation or by incorporating formic acid into the shampoo, lotion, or cream containing the active pediculicide.

### Summary of the Invention

The present invention provides an improved method of and composition for the removal of pediculosis nits (eggs) from the hair of individuals with pediculosis capitis (head lice). The method involves single or multiple applications of shampoos, rinses, creams, or lotions containing formic acid. The formic acid may be used in the same composition as/simultaneously with a chemical pediculicide, or it may be used separately for removing dead nits from the hair and scalp.

### Detailed Description of the Invention

In the practice of this invention formic acid is incorporated into topical products suitable for application to the hair and scalp, then applied and removed by washing after 5 to 15 minutes of contact with the hair. Products suitable for application to the hair and scalp into which formic acid may be incorporated for this use in concentrations of from 1% to 25% include shampoos, rinses, creams, gels, lotions and solutions. Concentrations of formic acid greater than 25% are very caustic and may cause severe damage to hair and scalp.

Although formic acid may be utilized successfully in connection of from 1% to 25% to remove nits, the preferred range which provides effective removal with minimal irritant effects is from 1% to 10%. Usually, a single application to the hair, left for 5 to 25 minutes, and them removed by washing is sufficient to remove all or most nits. However, additional applications may be required to remove remaining nits. The following examples illustrate the present invention.

### Example 1

A 12 year old female with pediculosis capitis utilized a 10% aqueous solution of formic acid following shampooing of her hair with lindane, an insecticide used to kill head lice. The formic acid solution was worked into the hair and allowed to remain for 5 minues, after which it was washed off in the shower. After drying and combing the hair, none of the over 50 nits that were present before treatment remained attached to the hair.

### Example 2

Formic acid was incorporated into a commercially available cream rinse to a concentration of about 1%. Three hairs with firmly attached nits were soaked in the formic acid containing rinse for 10 minutes in a glass plate. The three hairs were then rinsed in warm water and examined after air drying in the light. None of the hairs had a nit attached following this procedure.

### Example 3

Solutions of 1%, 5% and 25% formic acid were prepared in water and 3 different sets of three hairs to which nits (previously treated with lindane) were firmly attached were exposed to each concentration for 5 minutes each. The hairs were then washed, air dried and examined. All three nits were successfully removed from the hairs treated with 5% and 25% formic acid. One of the hairs treated in this fashion with 1% formic acid still retained a nit, while the other two hairs were nit-free.

### Example 4

Solutions of 1%, 2.5%, 5% and 25% formic acid were patch tested on the back of a 42 year female. Solutions were painted on previously marked one such by one inch areas of the back and left in place for 30 minutes before being removed. No irritation was observed in the areas treated with 1% or 2.5% formic acid; a ± erythema was noted in the patch treated area with 5% solution; a 1+ erythema was noted in the area where 25% formic acid had been applied and the subject complained of stinging and itching at that site.

While it is not fully understood how the formic acid accomplishes this result, it is believed that the formic acid reacts with and fractures or dissolves the cement holding the nit to the hair shaft.

While the foregoing has presented specific embodiments of the present invention, it is to be understood that these embodiments have been presented by way of example only.

## Claims

1. The use of formic acid for removing dead nits from the hair and scalp.

2. The use of formic acid as claimed in claim 1, wherein said formic acid is present in a topical composition in an amount not less than 1% by volume.

3. The use of formic acid as claimed in claim 2 in an amount not more than about 10% by volume.

4. A topical composition for killing lice and lice eggs and for removing nits from the hair and scalp of a human patient, said composition being characterized by a nit cement-dissolving amount of formic acid and a lice- and nit-killing amount of lindane, a pyrethrin compound, or a cholinesterase inhibitor.

5. The composition of claim 4, characterized in that said cholinesterase inhibitor is neostigmine or prostigmine.

6. The composition of claim 4 or claim 5, characterized in that formic acid is present in an amount not less than 1 percent by volume.

7. The composition of claim 6, characterized in that formic acid is present in an amount not more than about 10 percent by volume.

8. The use of a lice- and nit-killing amount of a chemically active pediculicide together with a nit cement-dissolving amount of formic acid for the manufacture of a topical composition for killing lice and lice eggs, and for removing nits from the hair and scalp of a human patient.

## Patentansprüche

1. Verwendung von Ameisensäure zur Entfernung toter Nissen aus Haar und Kopfhaut.

2. Verwendung von Ameisensäure gemäß Anspruch 1, worin die Ameisensäure in einer topischen Zusammensetzung in einer Menge von nicht weniger als 1 Vol.-% vorliegt.

3. Verwendung von Ameisensäure gemäß Anspruch 2 in einer Menge von nicht mehr als etwa 10 Vol.-%.

4. Topische Zusammensetzung zum Abtöten von Läusen und Läuse-Eiern und zur Entfernung von Nissen aus Haar und Kopfhaut eines menschlichen Patienten, gekennzeichnet durch eine Nissen-Zement-lösende Menge an Ameisensäure und eine Läuse- und Nissen-abtötende Menge an Lindan, einer Pyrethrin-Verbindung oder eines Cholinesterase-Inhibitors.

5. Zusammensetzung gemäß Anspruch 4, dadurch gekennzeichnet, daß der Cholinesterase-Inhibitor Neostigmin oder Prostigmin ist.

6. Zusammensetzung gemäß Anspruch 4 oder 5, dadurch gekennzeichnet, daß Ameisensäure in einer Menge von nicht weniger als 1 Vol.-% anwesend ist.

7. Zusammensetzung gemäß Anspruch 6, dadurch gekennzeichnet, daß Ameisensäure in einer Menge von nicht mehr als etwa 10 Vol.-% anwesend ist.

8. Verwendung einer Läuse- und-Nissen-abtötenden Menge eines chemisch aktiven Pediculicids zusammen mit einer Nissen-Zement-lösenden Menge an Ameisensäure für die Herstellung einer topischen Zusammensetzung zum Abtöten von Läusen und Läuse-Eiern und zur Entfernung von Nissen aus Haar und Kopfhaut eines menschlichen Patienten.

## Revendications

1. Utilisation d'acide formique pour éliminer les lentes mortes de la chevelure et du cuir chevelu.

2. Utilisation de l'acide formique suivant la revendication 1, dans laquelle ledit acide formique est présent dans une composition topique en quantité non inférieure à 1 %, en volume.

3. Utilisation de l'acide formique suivant la revendication 2, en quantité n'excédant pas 10% environ, en volume.

4. Composition topique destinée à tuer les poux et les oeufs de poux et à éliminer les lentes de la chevelure et du cuir chevelu d'un patient humain, ladite composition étant caractérisée par une quantité d'acide formique dissolvant le ciment des lentes, et par une quantité mortelle pour les poux et les lentes, de lindane, d'un composé pyréthrinoïde ou d'un inhibiteur de la cholinestérase.

5. Composition suivant la revendication 4, caractérisée en ce que ledit inhibiteur de la cholinestérase est la néostigmine ou la prostigmine.

6. Composition suivant la revendication 4 ou la revendication 5, caractérisée en ce que l'acide formique y est présent en quantité non inférieure à 1 pour cent, en volume.

7. Composition suivant la revendication 6, caractérisée en ce que l'acide formique y est présent en quantité n'excédant pas 10 pour cent environ, en volume.

8. Utilisation d'une quantité mortelle pour les poux et les lentes, d'un pédiculicide chimiquement actif, avec une quantité d'acide formique dissolvant le ciment des lentes, dans la fàbrication d'une composition topique destinée à tuer les poux et les oeufs de poux, et à éliminer les lentes, de la chevelure et du cuir chevelu d'un patient humain.
